# FASCICULE DE BREVET EUROPEEN

(11) **EP 2 061 390 B1**
(45) Date de publication et mention de la délivrance du brevet: **09.03.2016**
(21) Numéro de dépôt: 07823803.7
(22) Date de dépôt: 11.09.2007
(51) Int. Cl.: A61B 17/70, A61B 17/80, A61B 17/86

(54) **DISPOSITIF ORTHOPÉDIQUE IMPLANTABLE, EN PARTICULIER POUR RACHIS CERVICAL**
IMPLANTIERBARE ORTHOPÄDISCHE VORRICHTUNG, IM BESONDEREN FÜR DIE HALSWIRBELSÄULE
IMPLANTABLE ORTHOPAEDIC DEVICE, IN PARTICULAR FOR THE CERVICAL SPINE

(30) Priorité: 14.09.2006 FR 0608030
(43) Date de publication de la demande: 27.05.2009
(73) Titulaire: Newclip Technics, 44690 La Haye Fouassiere (FR)
(72) Inventeur: PODGORSKI, Jean-Pierre, 49230 Saint-Crespin-sur-Moine (FR); DEROUET, Guillaume, 44800 Saint-Herblain (FR)
(74) Mandataire: Michelet, Alain
(86) Numéro de dépôt international: PCT/FR2007/051907
(87) Numéro de publication internationale: WO 2008/031983

(56) Documents cités:
- US-A1- 2004 260 306

## Description

La présente invention concerne un nouveau dispositif orthopédique, du type constitué d'une plaque support munie d'au moins un orifice pour le passage d'une vis de fixation au matériau osseux de réception, adapté en particulier pour une implantation au niveau du rachis cervical d'un patient.

Dans le cas de fusion intervertébrale (arthrodèse), on utilise généralement un implant en forme de plaque permettant de stabiliser le rachis cervical et d'assurer le maintien en place du greffon osseux positionné par le praticien entre deux vertèbres, en remplacement du disque intervertébral extrait.
La plaque correspondante est munie de plusieurs orifices destinés chacun à recevoir une vis de fixation apte à s'ancrer dans le matériau osseux de réception. La forme et les dimensions de cette plaque, et aussi des vis associées, sont adaptées au site traité et aux contraintes d'implantation.
Très généralement, les plaques supports sont proposées aux praticiens en association avec un lot de vis de taille différente, dont seulement certaines sont utilisées en fonction du site d'implantation.

Pour une efficacité et une sécurité optimales, lors de l'implantation, le praticien applique un serrage particulier à chacune des vis, la plaque associée étant ainsi convenablement positionnée et maintenue contre les vertèbres.

La plupart des dispositifs orthopédiques actuels comporte des moyens de verrouillage ou de blocage des vis, afin d'empêcher ou tout au moins de limiter leurs mouvements de recul par rapport à la plaque associée, après remise en charge du montage (le desserrage correspondant des vis étant susceptible de gêner le patient, ou entraînant des risques majeurs de lésion).

Des dispositifs orthopédiques de ce type sont par exemple décrits dans les documents US-B-7 001 389 et US-2004/0260306. Dans ces documents, les vis de fixation comportent chacune une tête munie d'une rainure annulaire au sein de laquelle est prémonté un anneau de verrouillage élastique radialement. La plaque est quant à elle munie d'orifices dont la surface interne est munie d'une gorge délimitée par une bande de matière annulaire supérieure comprenant une face de dessus chanfreinée, et une face inférieure formant un méplat annulaire.
En pratique, chaque vis est implantée au travers de l'un des orifices de la plaque, et vissée jusqu'à ce que sa tête se verrouille automatiquement au sein de l'orifice associé. A cet effet, lorsque la tête de vis atteint la bordure supérieure de la gorge, son anneau élastique est compressé radialement par la surface de dessus chanfreinée précitée, puis il subit automatiquement une expansion au sein de cette gorge pour former la butée anti-recul ou anti-dévissage recherchée, avec le méplat annulaire mentionné ci-dessus.
Mais, avec de tels dispositifs, il est nécessaire d'équiper chaque vis avec un anneau de verrouillage, c'est-à-dire aussi bien les vis implantées que celles non employées, ce qui entraîne un surcoût non négligeable.
De plus, une fois les vis implantées, on ne peut pas les retirer sans endommager le matériel.

Un autre type de matériel du même genre est décrit dans le document FR-2 778 088. Ici, les moyens anti-recul des vis consistent en un tiroir monté coulissant sur la plaque support, apte à venir recouvrir partiellement les têtes de vis une fois celles-ci convenablement serrées.
Mais il s'agit là d'une structure relativement complexe. De plus, ce type d'implant nécessite un temps opératoire supplémentaire pour insérer ou retirer les vis, ainsi que du matériel complémentaire.
En outre, les moyens anti-recul ne sont forcément efficaces que sur un secteur bien déterminé de la périphérie de la vis.

Le document US-6 602 255 présente encore d'autres implants de ce genre. Dans une forme de réalisation possible, l'implant correspondant est constitué d'une plaque support munie d'orifices d'insertion des vis de fixation, au sein desquels est prévue une gorge d'accueil d'un anneau ouvert de verrouillage, de formes générales complémentaires. Sous la gorge équipée de son anneau de verrouillage, chaque orifice est muni d'une partie à surface périphérique sphérique femelle.
Les vis de fixation comportent une tête prolongée par un corps fileté. Ces têtes de vis sont équipées d'une protubérance ou excroissance annulaire périphérique à surface sphérique mâle, complémentaire de la surface sphérique femelle de l'orifice de plaque associé, ladite protubérance se raccordant avec la partie supérieure de ladite tête de vis par un méplat circulaire perpendiculaire à l'axe de vis.
Dans le cadre de la mise en place de l'implant, les vis de fixation sont serrées jusqu'à ce que la surface sphérique de leur protubérance annulaire vienne en contact avec la surface sphérique complémentaire de la plaque support. Avant ce contact, la protubérance annulaire est passée à force à travers l'anneau fendu en réalisant l'expansion radiale de ce dernier au sein de sa gorge de réception. Après que le méplat circulaire supérieur de cette protubérance ait passé le niveau de l'anneau fendu, celui-ci reprend automatiquement sa position repos pour former l'organe de verrouillage recherché (par contact plan entre le méplat annulaire de la vis et la face inférieure de l'anneau fendu).
Les surfaces de contact sphérique entre les orifices de plaque et les têtes de vis offrent au praticien une possibilité de réglage angulaire des vis.

Mais, étant donnée la structure de cet implant, lorsque l'axe de la vis n'est pas confondu avec l'axe de son orifice de réception, le contact entre le méplat annulaire des têtes de vis et l'anneau de verrouillage n'est pas optimal, ce qui minimise la surface d'appui s'opposant au recul de la vis.
En outre, le démontage des vis après implantation n'est pas très facile à réaliser et nécessite du matériel complémentaire particulier. Cela entraîne un surcoût en terme de matériel et aussi un temps opératoire supplémentaire.

La demanderesse a développé une structure alternative d'implant, ayant l'intérêt d'être simple, fiable et efficace. En outre, cette structure est facilement réversible ; les mêmes plaques et les mêmes vis peuvent être repositionnées après un premier essai, ceci sans nécessiter la mise en oeuvre de matériel complémentaire.

A cet effet, l'implant conforme à l'invention se compose, d'une part, d'une structure support de type plaque qui est délimitée par une face de dessus et par une face de dessous, et qui est munie d'au moins un orifice traversant, et d'autre part, d'au moins une vis composée d'une tête de vis et d'un corps de vis. Ledit orifice traversant est conformé pour permettre le passage de ladite vis, le contact entre ladite tête de vis et ladite plaque étant réalisé par l'intermédiaire de surface sphériques complémentaires. Ce dispositif orthopédique est encore du type comportant des moyens de verrouillage rapportés, aptes à empêcher, ou tout au moins limiter, le recul de la vis une fois sa tête convenablement en appui sur la plaque, ces moyens de verrouillage étant constitués d'une gorge annulaire ménagée sur une partie de la hauteur de la surface interne de l'orifice de plaque, délimitée par une collerette annulaire supérieure et par une collerette annulaire inférieure, au sein de laquelle gorge est rapporté un anneau métallique fendu à capacité de déformation élastique radiale dont, au repos, - d'une part la bordure interne présente un diamètre inférieur à celui dudit orifice, et - d'autre part, la bordure externe présente un diamètre inférieur à celui du fond de ladite gorge annulaire. D'autre part, la tête de vis est du type comportant une protubérance annulaire divergente depuis le corps de vis jusqu'à une rainure annulaire ; cette protubérance est conformée pour permettre, lorsque ladite tête de vis progresse au sein de l'orifice associé, l'expansion radiale de l'anneau élastique de verrouillage au sein de sa gorge de réception, puis, en phase terminale de vissage, le logement dudit anneau élastique au sein de ladite rainure annulaire de vis, par rétrécissement radial élastique, de sorte à constituer lesdits moyens de verrouillage anti-recul.

Dans le cadre d'une telle structure, l'implant conforme à l'invention est caractérisé par le fait que :
a- l'orifice de la plaque support est délimité, depuis la face de dessus de ladite plaque support, jusqu'à sa face de dessous, successivement par :
   a1- une surface de contact sphérique femelle ménagée au niveau de la bordure interne de la collerette supérieure de gorge,
   a2- la gorge de réception de l'anneau de verrouillage, délimitée par un épaulement annulaire supérieur, une surface de fond cylindrique ou sensiblement cylindrique et un épaulement annulaire inférieur, lesdits épaulements supérieur et inférieur s'étendant perpendiculairement à l'axe de l'orifice de plaque et leur distance a définissant l'épaisseur de ladite gorge, et
   a3- la bordure interne de la collerette inférieure de gorge, cette bordure interne étant surdimensionnée par rapport à la portion de la vis destinée à venir en regard.
b- la tête de vis comporte, depuis son extrémité libre jusqu'au corps de vis, successivement :
   b1 - une surface de contact sphérique mâle, complémentaire de la surface sphérique femelle de la plaque support,
   b2 - la rainure annulaire d'accueil de l'anneau de verrouillage, délimitée par un épaulement perpendiculaire à l'axe de vis, une surface de fond cylindrique ou sensiblement cylindrique et une portion de forme générale tronconique divergente, et
   b3 - la protubérance annulaire aboutissant au corps de vis, laquelle protubérance présente une forme générale tronconique divergente depuis ledit corps de vis jusqu'à ladite rainure, son diamètre minimal étant inférieur à celui de la bordure interne de l'anneau de verrouillage et son diamètre maximal étant compris entre celui de ladite bordure interne d'anneau et le diamètre minimal de l'orifice de plaque.
c- en outre, l'anneau élastique de verrouillage a une épaisseur b inférieure à l'épaisseur a de sa gorge d'accueil, pour lui conférer au moins un degré de liberté axial à l'intérieur de cette gorge.

Cette combinaison de caractéristiques permet d'obtenir un verrouillage efficace des vis après serrage.
L'anneau de verrouillage, monté « flottant » au sein de sa gorge d'accueil, permet de s'opposer efficacement au mouvement de recul de la vis, avec une surface d'appui sur la vis qui est optimisée de par sa faculté à s'auto-aligner par rapport à l'axe de ladite vis, et aussi par sa capacité à se déformer au contact de la surface supérieure de la gorge d'accueil.
De plus, la portion tronconique divergente de la rainure ménagée dans la tête de vis autorise, en cas de besoin, le démontage de la vis par le praticien, sans endommager la plaque ni l'anneau fendu, et en utilisant uniquement le matériel de mise en place de la vis (manoeuvré en contre rotation).

Selon une autre particularité, le diamètre du fond de la gorge annulaire du ou des orifices de la plaque support est au moins égal au diamètre maximal de la protubérance annulaire de la tête de vis, additionné de deux fois la largeur de l'anneau élastique associé, ladite largeur d'anneau correspondant à la distance séparant ses bordures interne et externe.

De préférence, l'anneau élastique est délimité par deux surfaces annulaires planes parallèles, dont l'écartement définit l'épaisseur b dudit anneau. En outre, le diamètre de la bordure interne de l'anneau, au repos, est avantageusement supérieur à celui du fond de la rainure annulaire de la tête de vis, pour faciliter l'insertion de la vis sur l'anneau et optimiser l'auto-alignement de l'axe de l'anneau par rapport à l'axe de la vis.

Encore de préférence, l'épaisseur b de l'anneau élastique est inférieure à la hauteur de la rainure annulaire de la tête de vis.

Selon une forme de réalisation préférentielle, la bordure interne de l'anneau élastique a une section en forme générale de Vé convergent vers l'intérieur, définissant deux surfaces inclinées convergentes, l'une de dessus et l'autre de dessous.
Cette particularité permet de faciliter le montage de la vis, et son démontage éventuel.

Selon une variante de réalisation particulièrement intéressante, la bordure interne de la collerette supérieure de la plaque support comporte un filetage cylindrique ménagé dans la surface de contact sphérique femelle.

Dans le cadre de cette variante de réalisation, au moins l'une des vis de fixation proposée au praticien, en complément de la structure de vis décrite ci-dessus, comprend, depuis son extrémité libre jusqu'au corps de vis :
- une partie d'extrémité cylindrique, munie d'un filetage externe complémentaire du filetage ménagé sur la bordure interne de la collerette supérieure de la plaque support,
- une rainure annulaire d'accueil de l'anneau élastique, et
- une protubérance annulaire.
L'épaulement annulaire supérieur de ladite rainure est adapté pour venir prendre appui sur la face en regard de l'anneau élastique, lui-même en appui contre la collerette inférieure de la plaque support, pour obtenir un verrouillage monobloc vis/plaque. Cette particularité offre au praticien une alternative de vissage de la plaque support, utilisée selon les besoins en fonction du site d'implantation.

Toujours selon cette variante de réalisation, la bordure externe de l'anneau de verrouillage comporte avantageusement un filetage complémentaire de celui de ladite collerette supérieure, ce qui permet le positionnement dudit anneau de verrouillage au sein de sa gorge de réception, après vissage au travers dudit filetage de plaque.
Cette particularité facilite le positionnement de l'anneau au sein de sa gorge de réception.

L'invention sera encore illustrée, sans être aucunement limitée, par la description suivante de plusieurs formes de réalisations possibles, données uniquement à titre d'exemples et représentées sur les dessins annexés dans lesquels :
- la figure 1 est une vue schématique et fonctionnelle d'un dispositif orthopédique conforme à l'invention, dans laquelle la vis d'ancrage est prépositionnée convenablement pour son implantation au travers de l'orifice d'une plaque support dessinée partiellement ;
- la figure 2 est une vue schématique et fonctionnelle du dispositif orthopédique selon la figure 1, dans laquelle la vis arrive en phase terminale de serrage ;
- la figure 3 est toujours une vue schématique et fonctionnelle du dispositif orthopédique selon les figures 1 et 2, dans laquelle la tête de la vis est convenablement verrouillée au sein de l'orifice de la plaque support ;
- la figure 4 est une vue en perspective de la plaque support d'une forme de réalisation possible d'un dispositif orthopédique tel qu'illustré schématiquement sur les figures 1 à 3;
- la figure 5 est une vue en perspective d'un anneau de verrouillage destiné à être prémonté dans la plaque support de la figure 4 ;
- la figure 6 est une vue de côté de l'une des vis d'ancrage employées pour la fixation de la plaque de la figure 4 ;
- la figure 7 est une vue générale et en perspective de la vis de la figure 6, convenablement montée au travers de l'un des orifices de réception de la plaque support de la figure 4 ;
- la figure 8 est une vue en coupe transversale du dispositif orthopédique de la figure 7, selon un plan de coupe passant par l'axe de la vis, cela pour montrer le verrouillage de cette vis sur la plaque support par les moyens anti-recul ;
- la figure 9 est une vue agrandie d'une partie de la figure 8, montrant l'anneau de verrouillage positionné dans la gorge de la plaque support et dans la rainure de la tête de vis ;
- la figure 10 est une vue en perspective, avec coupe transversale, d'une variante de réalisation d'un dispositif orthopédique conforme à l'invention ;
- la figure 11 montre en perspective l'anneau de verrouillage, isolé, utilisé dans le cadre du mode de réalisation d'implant de la figure 10 ;
- la figure 12 est une vue en perspective d'une vis particulière, susceptible d'être proposée au praticien en association avec les vis de fixation décrites en liaison avec le mode de réalisation des figures 1 à 9, dans le cadre de la variante illustrée sur la figure 10.

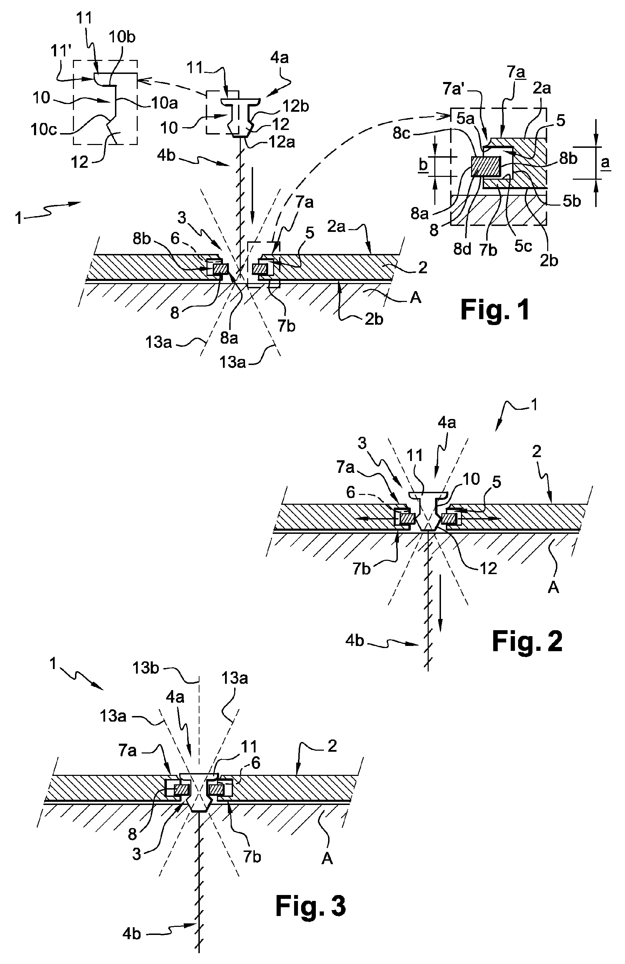

Tel que représenté schématiquement sur les figures 1 à 3, le dispositif orthopédique 1 se compose d'une structure support 2 (ou structure de réception) en forme de plaque, dans laquelle est ménagé au moins un orifice circulaire 3 (s'étendant entre ses faces de dessus 2a et de dessous 2b). Chacun de ces orifices 3 est apte à recevoir une vis 4 pour la fixation de la plaque 2 sur le matériau osseux A.

Ce dispositif orthopédique 1 est par exemple destiné à être implanté entre deux vertèbres du rachis cervical, en cas d'arthrodèse. Il a alors pour fonction de stabiliser les deux vertèbres l'une par rapport à l'autre et d'empêcher l'expulsion du greffon intervertébral positionné par le praticien.

Le dispositif orthopédique 1 comporte également des moyens pour le verrouillage de chaque vis 4 par rapport à la plaque 2, en particulier pour empêcher leur desserrage une fois qu'elles sont convenablement implantées dans le matériau osseux de réception, de manière à empêcher la migration des vis et du greffon.
Ces moyens de verrouillage sont prémontés au sein des orifices 3 de la plaque 2, avant mise en place des vis 4.

En l'occurrence, l'orifice 3 comporte une gorge annulaire 5, ménagée sur une partie de la hauteur de sa surface interne 6 (entre les faces de dessus 2a et de dessous 2b de la plaque 2), délimitée par des collerettes de dessus 7a et de dessous 7b. On note que la bordure interne 7a' de la collerette de dessus 7a a une forme générale sphérique femelle, conformée pour coopérer avec une partie complémentaire ménagée sur la tête de vis 4a, comme décrit plus en détail par la suite.

La gorge 5 est ainsi délimitée :
- par un épaulement annulaire circulaire 5a, dans le prolongement de la bordure interne sphérique 7a' de la collerette de dessus 7a,
- par une surface de fond 5b cylindrique ou sensiblement cylindrique, et
- par un épaulement annulaire inférieur 5c.
Ces épaulements supérieur 5a et inférieur 5c sont parallèles et s'étendent perpendiculairement à l'axe de l'orifice 3 ; la distance a qui les sépare définit l'épaisseur de la gorge 5.

Un anneau fendu 8 à capacité de déformation élastique radiale est prémonté au sein de cette gorge annulaire 5 pour constituer les moyens de verrouillage de la vis 4.

Au repos, figure 1, on peut voir que cet anneau élastique 8 comporte une bordure interne 8a dont le diamètre est inférieur à celui de l'orifice 3, et une bordure externe 8b dont le diamètre est compris entre celui de l'orifice 3 et celui du fond 5b de la gorge annulaire 5.
Cet anneau élastique 8 s'étend ainsi, à la fois, partiellement au sein de la gorge annulaire 5 et dans l'encombrement de l'orifice 3.

L'anneau élastique 8 est en outre délimité par deux surfaces planes annulaires parallèles, l'une de dessus 8c et l'autre de dessous 8d, dont l'écartement définit l'épaisseur b dudit anneau 8.
On remarque que cette épaisseur b de l'anneau élastique 8 est inférieure à celle a de la gorge annulaire associée.

La vis 4 comporte une tête 4a conformée pour coopérer avec des moyens de vissage manoeuvrés par le praticien, et un corps de vis 4b muni d'un filet permettant son vissage dans le matériau osseux de réception.

Cette vis 4 est particulière du fait que sa tête 4a comporte une rainure annulaire périphérique 10 avec, de part et d'autre :
- du côté opposé au corps de vis 4b, une partie d'extrémité libre 11 dont la bordure périphérique 11' a une forme générale sphérique mâle, et
- du côté du corps de vis 4b, une protubérance 12, s'étendant entre ledit corps de vis 4b et la rainure annulaire 10 précitée.
La bordure périphérique sphérique supérieure 11' de la tête de vis 4a forme une surface de contact complémentaire à la bordure interne 7a' de la collerette supérieure 7a de la plaque support 2.

La rainure annulaire 10 est destinée à recevoir l'anneau élastique 8 de la plaque 2, lorsque la vis 4 atteint sa phase terminale de vissage. Cette rainure annulaire 10 présente une section de forme générale trapézoïdale avec une surface de fond 10a cylindrique ou sensiblement cylindrique, prolongée par un épaulement annulaire supérieur 10b, du côté de la partie d'extrémité libre 11 de la tête de vis 4a, et par une surface tronconique 10c divergente vers le corps de vis 4b.
L'épaulement annulaire 10b s'étend perpendiculairement à l'axe de la vis 4 ; la surface tronconique 10c est inclinée d'un angle compris entre 30 et 60° par rapport à l'axe de ladite vis 4.
La surface de fond cylindrique 10a de la rainure 10 a un diamètre légèrement inférieur au diamètre (au repos) de la bordure interne 8a de l'anneau élastique 8, et une hauteur supérieure à l'épaisseur b dudit anneau 8.

La protubérance 12 est conformée pour permettre, lors de la phase terminale de vissage de la vis 4, l'expansion radiale de l'anneau élastique 8 au sein de sa gorge de réception 5 (figure 2), puis le logement dudit anneau élastique 8 au sein de la rainure annulaire 10 par rétrécissement radial élastique (figure 3), pour verrouiller la vis 4 par rapport à la plaque 2.
On comprend bien, qu'en cas de léger dévissage de la vis 4, la surface tronconique 10c de la rainure 10 va venir en appui contre l'anneau élastique 8, ce dernier empêchant la poursuite du dévissage.

La protubérance 12 de la tête de vis 4a présente une forme générale tronconique, divergente depuis le corps de vis 4b jusqu'à la rainure annulaire 10. Cette protubérance comporte principalement :
- une première extrémité 12a du côté du corps de vis 4b, dont le diamètre est inférieur à celui de la bordure interne 8a de l'anneau 8 au repos, et
- une seconde extrémité 12b, du côté de la rainure 10, dont le diamètre est supérieur à celui de cette bordure interne d'anneau 8a.

En pratique, le corps de vis 4b est inséré au travers de l'orifice 3 et de son anneau élastique 8 associé ; l'inclinaison de la vis 4 par rapport à la plaque 2 est choisie par le praticien en fonction de la configuration du site d'implantation, ceci dans le domaine d'orientation admissible délimité par les axes 13a. La position médiane, perpendiculaire à la plaque support 2, est représentée par l'axe 13b, ce dernier étant coaxial à l'axe de l'orifice 3 de la plaque 2.

La forme et les dimensions de la gorge 5 et de l'anneau 8 procurent à ce dernier au moins un degré de liberté axiale dans ladite gorge 5, supérieure à celui d'un simple jeu fonctionnel, permettant ainsi l'autocentrage respectif de la vis 4 et de l'anneau 8, ceci quelle que soit l'orientation admissible de l'axe de la vis 4 par rapport à l'axe de l'orifice 3.

La vis 4 est ensuite serrée par le praticien dans le matériau osseux de réception A, jusqu'à ce que la partie sphérique 11' de sa tête 4a vienne prendre appui sur la surface sphérique complémentaire 7a' de la collerette de dessus 7a de l'orifice 3 (figure 3). Ces deux surfaces de contact sphérique complémentaires 7a', 11' assurent alors un appui optimal de la tête de vis 4a sur la plaque de réception 2, cela quelle que soit l'orientation de la vis par rapport à l'axe de l'orifice 3, dans le secteur angulaire admissible précité.
Pour autoriser un tel réglage angulaire de la vis 4, comme on peut le voir sur la figure 3, la protubérance 12 de la tête de vis 4a a un encombrement légèrement inférieur à celui de la bordure interne de la collerette de dessous 7b en regard. L'épaisseur de l'anneau 8, inférieure à celle de la rainure annulaire 10, contribue également à cette possibilité de réglage angulaire, et optimise l'auto-centrage anneau/vis précité.

Lors de la phase terminale de vissage (figure 2), la tête de vis 4 pénètre au travers de l'orifice 3.
La protubérance 12 de la tête de vis 4a vient interagir avec la bordure interne 8a de l'anneau élastique 8, ce dernier subissant alors une expansion radiale (figure 2). A cet effet, le diamètre du fond de la gorge 5 est au moins égal au diamètre maximal 12b de la protubérance 12 additionné de deux fois la largeur de l'anneau 8, cette largeur correspondant à la distance entre ses bordures interne 8a et externe 8b.
L'anneau élastique 8 chemine progressivement sur la longueur de la protubérance 12 avec l'avancement de la tête 4a, jusqu'à la rainure annulaire 10 au sein de laquelle il se rétreint automatiquement radialement, reprenant son état « repos » (figure 3). Dans cette dernière configuration, l'anneau 8 constitue un moyen assurant un verrouillage structurel entre la tête de vis 4a et l'orifice 3 de la plaque 2. Cet anneau forme en effet une butée entre la collerette de dessus 7a de l'orifice 3 et l'extrémité inférieure 10c de la rainure annulaire 10, via l'anneau 8 ; ce blocage permet de prévenir, ou tout au moins de limiter grandement, les phénomènes de recul ou de dévissage de la vis 4 lors du retour en charge de l'implant.

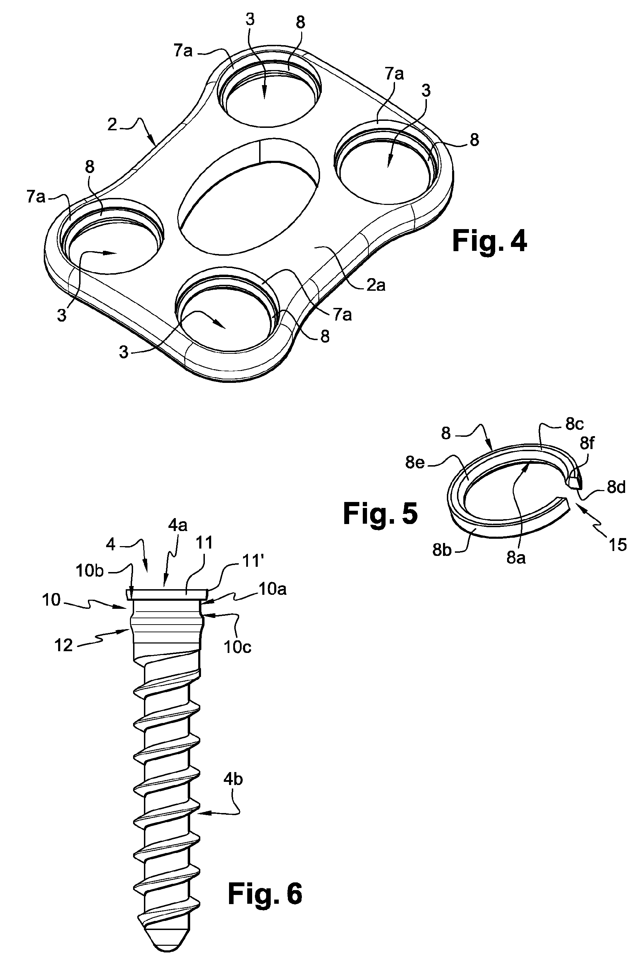

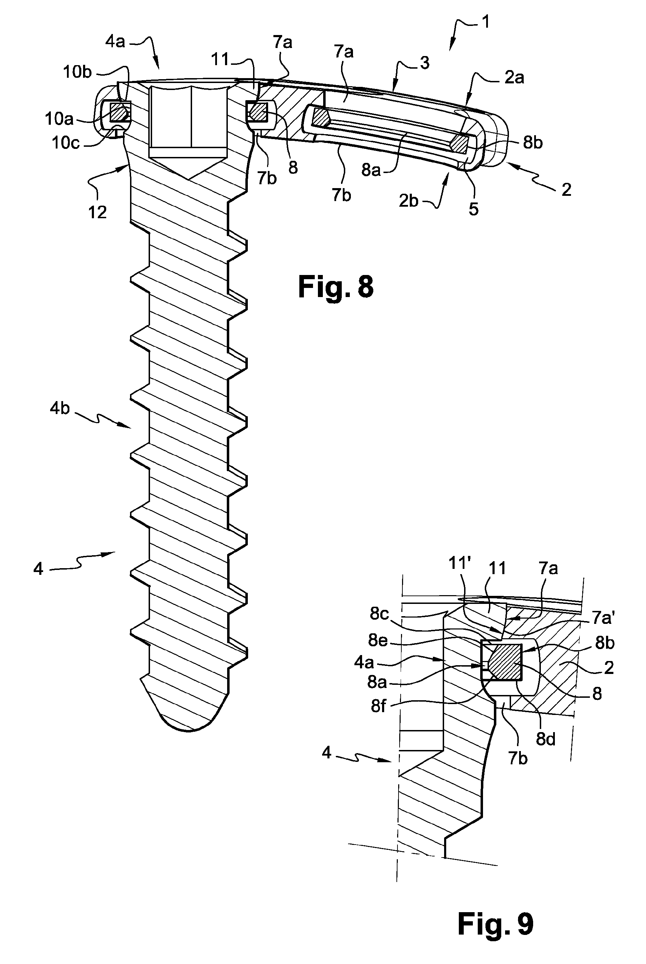

Une forme de réalisation particulière du dispositif orthopédique 1, selon les figures schématiques 1 à 3, est représentée sur les figures 4 à 9.

Sur la figure 4, on retrouve une plaque support 2 dans laquelle sont ménagés quatre orifices 3 munis chacun d'un anneau déformable radialement 8, et au travers de chacun desquels une vis d'ancrage 4 est destinée à transiter pour être implantée dans le matériau osseux de réception.

La plaque support 2 est réalisée par exemple dans un matériau de type titane TA6V, d'épaisseur de l'ordre de 2 mm. Ses orifices 3 ont par exemple un diamètre de l'ordre de 5 mm ; ils présentent chacun une gorge annulaire 5 d'une hauteur de l'ordre de 1,1 mm et d'un diamètre périphérique de 0,7 mm.

Les anneaux de verrouillage 8 logés dans les gorges 5 sont réalisés en matériau métallique de type titane TA6V, et comportent chacun une ouverture 15 (figure 5) conférant leur capacité de déformation élastique radiale.
Ils présentent chacun des bordures interne 8a et externe 8b dont le diamètre est respectivement de 4,7 mm et 6 mm. Leur épaisseur est de 0,7 mm.

Comme on peut le voir sur la figure 5, ces anneaux élastiques 8 ont une bordure interne 8a dont la section est de forme générale triangulaire, avec une pointe centrée, orientée intérieurement. Cette forme particulière définit deux surfaces inclinées convergentes, l'une supérieure 8e et l'autre inférieure 8f qui favorisent la capacité de déformation de l'anneau, et optimisent aussi le domaine d'orientation admissible de l'axe de la vis 4 par rapport à l'axe de l'orifice 3.
Les deux surfaces inclinées correspondantes favorisent, pour l'une 8e le montage de la vis 4, par coopération avec la protubérance 12, et pour l'autre 8f le démontage de la vis 4, par coopération avec la surface tronconique 10c de la rainure 10.
De plus, cette pointe en Vé limite les surfaces de contact avec la tête de vis 4a, ce qui contribue à limiter les frottements et optimise le transfert des efforts de déformation.

D'autre part, figure 6, on retrouve la vis 4 comportant :
- d'une part, une tête de vis 4a munie de la partie d'extrémité 11 avec son contour sphérique 11' et de la protubérance 12, séparées par la rainure annulaire 10, et
- d'autre part, un corps de vis fileté 4b.

Comme décrit ci-dessus, la rainure annulaire 10 a une section générale trapézoïdale avec une surface de fond cylindrique 10a prolongée par l'épaulement annulaire supérieur 10b (côté extrémité libre 11), et par la surface tronconique inférieure 10c (côté protubérance 12).
Tel qu'indiqué auparavant, la surface tronconique 10c a pour fonction de former une sorte de rampe d'expansion radiale pour l'anneau élastique 8 logé dans la rainure 10, cela en particulier pour permettre le démontage de la vis 4 par une simple opération de dévissage volontaire.

A titre indicatif, la rainure annulaire 10 comporte une surface de fond 10a présentant un diamètre de l'ordre de 4,5 mm, et une hauteur de 1 mm ; l'inclinaison de la surface divergente 10c est de l'ordre de 45° par rapport à l'axe de la vis.

Par ailleurs, la protubérance 12 présente une forme générale tronconique dont le diamètre minimal est de 4,5 mm, et celui maximal de l'ordre de 5 mm, avec une hauteur de l'ordre de 1,5 mm.

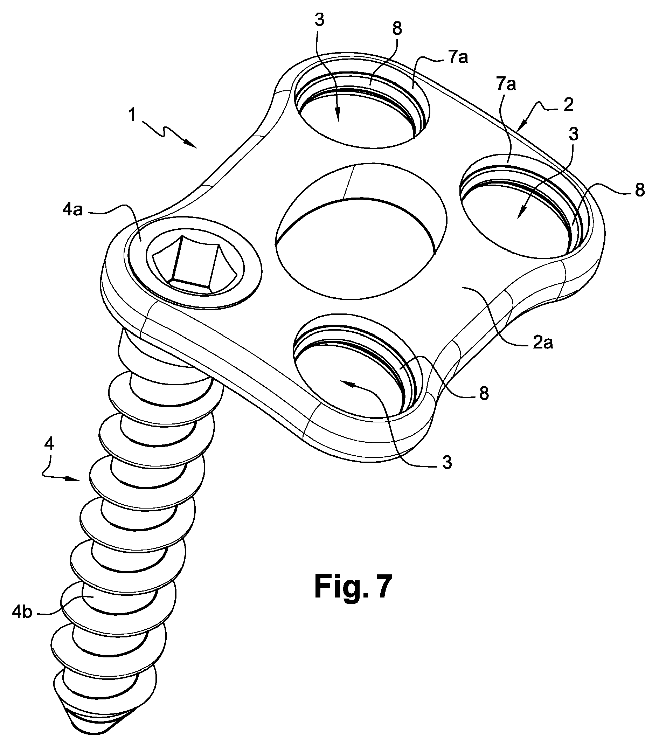

Comme expliqué auparavant, l'implantation du dispositif orthopédique 1 décrit ci-dessus en relation avec les figures 4 à 6 consiste à passer le corps de vis 4a d'une vis 4 au travers de l'un des orifices 3 de la plaque 2, et à le visser dans le matériau osseux jusqu'à ce que la tête 4a se place au sein de l'orifice 3 précité où elle est verrouillée automatiquement par l'anneau élastique 8 (figures 7, 8 et 9).
A titre indicatif, dans cette forme de réalisation, l'amplitude admissible de l'axe de la vis 4 par rapport à l'axe de l'orifice 3 est comprise entre 10 et 20°.

Le dispositif orthopédique selon l'invention permet un verrouillage simple et efficace de la vis 4 lorsqu'elle est convenablement positionnée par le praticien au sein du matériau osseux de réception. Cette vis est démontable. En outre, le fait de positionner les anneaux de verrouillage sur la plaque support avant la mise en place des vis de fixation, permet de simplifier la structure du jeu de pièces d'implantation proposées au praticien.

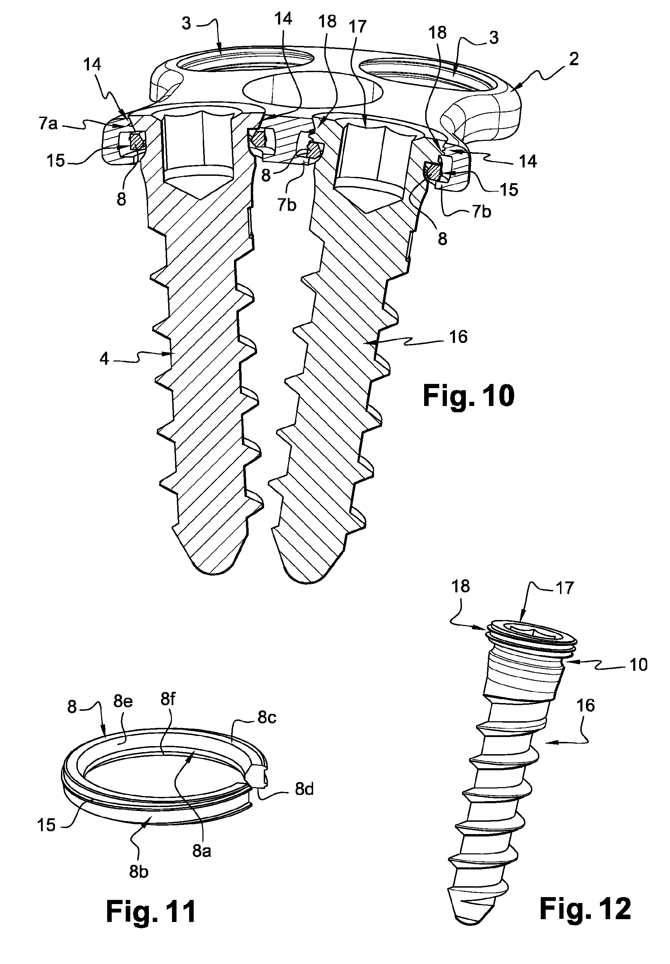

Les figures 10 à 12 illustrent une variante de réalisation de l'implant.

Comme on peut le voir sur la figure 10, cette variante de réalisation diffère de celle illustrée sur les figures 4 à 9 par la présence d'un filetage cylindrique 14 ménagé dans la surface de contact sphérique 7a' de la plaque 2, coaxialement à l'orifice 3.

La bordure externe 8b de l'anneau de verrouillage 8 comporte alors avantageusement un filetage 15 complémentaire audit filetage de plaque 14.
De la sorte, le montage de l'anneau 8 sur la plaque support 2 est réalisé par son vissage dans la collerette supérieure de plaque 7a, jusqu'à ce qu'il atteigne la gorge de réception 5 au sein de laquelle il chute et se positionne de manière « flottante ».
Ce positionnement de l'anneau 8 par vissage s'avère techniquement plus facile à mettre en oeuvre, par rapport à une insertion par simple encastrement (en particulier du fait des petites dimensions de pièces en présence).

La figure 11 montre l'anneau de verrouillage 8 correspondant, de manière agrandie, équipé du filetage 15 sur sa bordure externe 8b.

Le filetage 14 ménagé dans la couronne supérieure 7a de la plaque support 2 est réalisé en creux et ne gêne aucunement le positionnement d'une vis de fixation 4 telle que décrite précédemment, comme on peut le voir sur la partie gauche de la figure 10.

D'autre part, la présence du filetage 14 est mise à profit pour proposer au praticien une ou plusieurs vis complémentaires 16 (dans le lot de vis proposées avec un jeu de plaques), de structure différente des vis de fixation 4 décrites ci-dessus, utilisable(s) en particulier pour réaliser un assemblage monobloc vis/plaque.

Une forme de réalisation possible d'une telle vis 16 est illustrée, en application, sur la partie droite de la figure 10, et isolément sur la figure 12.

Cette vis 16 est très similaire aux vis 4 décrites ci-dessus, la seule différence étant le remplacement de la partie sphérique d'extrémité 11-11' par une partie d'extrémité cylindrique 17 équipée d'un filetage périphérique externe 18 complémentaire au filetage 14 de plaque.

Comme illustré sur la partie droite de la figure 10, cette vis 16 peut être vissée dans l'orifice 3 de la plaque support 2 équipée d'un anneau de verrouillage 8. En fin de phase de vissage, la partie cylindrique d'extrémité 17 de la vis 16 se visse sur le filetage 14 de la collerette supérieure 7a, jusqu'à ce que l'épaulement annulaire supérieur 10b de la rainure 10 vienne prendre appui sur l'anneau de verrouillage 8, lui-même en appui sur la collerette inférieure 7b.
De préférence, le pas du filetage 18 est identique au pas du filetage du corps de vis ; mais il peut aussi être différent, en fonction des effets mécaniques recherchés.
On obtient alors un assemblage monobloc vis/plaque muni d'un moyen anti-recul 8.

On comprend que cette variante de réalisation autorise l'application de deux techniques de fixation de la plaque support, par utilisation des vis de fixation 4 et/ou 16, ce qui permet, en fonction des attentes chirurgicales, d'obtenir un système contraint ou semi-contraint.

On notera, dans le cadre de cette variante de réalisation avec filetage de plaque 14, que l'anneau de verrouillage 8 peut tout-à-fait correspondre à celui décrit en liaison avec les figures 4 à 9, c'est-à-dire être dépourvu du filetage extérieur 15.

## Revendications

1. Dispositif orthopédique, en particulier implantable au niveau du rachis cervical d'un patient, lequel dispositif orthopédique (1) se compose, d'une part, d'une structure support (2) de type plaque, qui est délimitée par une face de dessus (2a) et par une face de dessous (2b) et qui est munie d'au moins un orifice traversant (3), et d'autre part, d'au moins une vis (4) composée d'une tête de vis (4a) et d'un corps de vis (4b), ledit orifice traversant (3) étant conformé pour permettre le passage de ladite vis (4), le contact entre ladite tête de vis (4a) et ladite plaque (2) étant réalisé par l'intermédiaire de surfaces sphériques complémentaires (11', 7a'), lequel dispositif orthopédique (1) comporte encore des moyens de verrouillage rapportés, aptes à empêcher ou tout au moins limiter le recul de ladite vis (4) une fois sa tête (4a) convenablement en appui sur ladite plaque (2), lesquels moyens de verrouillage sont constitués d'une gorge annulaire (5), ménagée sur une partie de la hauteur de la surface interne (6) dudit orifice (3), délimitée par une collerette annulaire supérieure (7a) et par une collerette annulaire inférieure (7b), ladite gorge (5) étant délimitée par un épaulement annulaire supérieur (5a), une surface de fond (5b) cylindrique ou sensiblement cylindrique et un épaulement annulaire inférieur (5c), lesdits épaulements supérieur (5a) et inférieur (5c) s'étendant perpendiculairement à l'axe de l'orifice (3) et leur distance (a) définissant l'épaisseur de ladite gorge (5), et au sein de laquelle gorge (5) est rapporté un anneau métallique fendu (8) à capacité de déformation élastique radiale dont, au repos, - d'une part, la bordure interne (8a) présente un diamètre inférieur à celui dudit orifice (3), et - d'autre part, la bordure externe (8b) présente un diamètre inférieur à celui du fond (5b) de ladite gorge annulaire (5), l'épaisseur (b) dudit anneau élastique (8) étant inférieure à l'épaisseur (a) de ladite gorge (5) dans laquelle il est logé, pour lui conférer au moins un degré de liberté axial à l'intérieur de ladite gorge (5), ladite tête de vis (4a) comportant une protubérance annulaire (12) divergente depuis ledit corps de vis (4b) jusqu'à une rainure annulaire (10), délimitée par un épaulement (10b) perpendiculaire à l'axe de la vis (4), une surface de fond (10a), cylindrique ou sensiblement cylindrique, et une portion (10c), ladite protubérance (12) étant conformée pour permettre, lorsque ladite tête (4a) progresse au sein dudit orifice (3) associé, l'expansion radiale dudit anneau élastique (8) au sein de sa gorge de réception (5), puis, en phase terminale de vissage, le logement dudit anneau élastique (8) au sein de ladite rainure annulaire (10), par rétrécissement radial élastique, de sorte à constituer lesdits moyens de verrouillage anti-recul,
**caractérisé en ce que** :
a- l'orifice (3) de la plaque support (2) est délimité depuis la face de dessus (2a) de ladite plaque (2) jusqu'à sa face de dessous (2b), successivement, par :
- une surface de contact sphérique femelle (7a') ménagée au niveau de la bordure interne de ladite collerette supérieure (7a),
- la bordure interne de ladite collerette inférieure (7b), cette bordure interne étant surdimensionnée par rapport à la portion de ladite vis (4) destinée à venir en regard,
b- ladite tête de vis (4a) comporte, depuis son extrémité libre jusqu'audit corps de vis (4b), successivement :
b1 - une surface de contact sphérique mâle (11'), complémentaire de la surface sphérique femelle (7a') de la plaque support (2),
b2 - ladite rainure annulaire (10) délimitée par un épaulement (10b) perpendiculaire à l'axe de la vis (4), une surface de fond (10a), cylindrique ou sensiblement cylindrique, et une portion (10c) de forme générale tronconique divergente, et
b3 - ladite protubérance annulaire (12) aboutissant audit corps de vis (4b), laquelle protubérance (12) présente une forme générale tronconique divergente depuis ledit corps de vis (4b) jusqu'à ladite rainure (10), son diamètre minimal étant inférieur à celui de la bordure interne (8a) dudit anneau élastique (8) et son diamètre maximal étant compris entre celui de ladite bordure interne (8a) dudit anneau (8) et le diamètre minimal dudit orifice (3).

2. Dispositif orthopédique selon la revendication 1, **caractérisé en ce que** le diamètre du fond de la gorge annulaire (5) de l'orifice (3) est au moins égal au diamètre maximal de la protubérance (12), additionné de deux fois la largeur de l'anneau élastique (8) associé, ladite largeur de l'anneau (8) correspondant à la distance séparant ses bordures interne (8a) et externe (8b).

3. Dispositif orthopédique selon l'une quelconque des revendications 1 ou 2, **caractérisé en ce que** l'anneau élastique (8) est délimité par deux surfaces annulaires planes parallèles (8c) et (8d) dont l'écartement définit l'épaisseur (b) dudit anneau (8).

4. Dispositif orthopédique selon l'une quelconque des revendications 1 à 3, **caractérisé en ce que** l'anneau élastique (8) a une bordure interne (8a) dont, au repos, le diamètre est supérieur à celui du fond (10a) de la rainure annulaire (10) de la tête de vis (4a).

5. Dispositif orthopédique selon l'une quelconque des revendications 1 à 4, **caractérisé en ce que** l'anneau élastique (8) a une épaisseur (b) inférieure à la hauteur de la rainure annulaire (10) de la tête de vis (4a).

6. Dispositif orthopédique selon l'une quelconque des revendications 1 à 5, **caractérisé en ce que** l'anneau élastique (8) comporte une bordure interne (8a) dont la section a une forme générale de Vé convergent vers l'intérieur, définissant deux surfaces inclinées convergentes, l'une de dessus (8e) et l'autre de dessous (8f).

7. Dispositif orthopédique selon l'une quelconque des revendications 1 à 6, **caractérisé en ce que** la bordure interne de la collerette supérieure (7a) de la plaque support (2) comporte un filetage cylindrique (14) ménagé dans la surface de contact sphérique femelle (7a').

8. Dispositif orthopédique selon la revendication 7, **caractérisé en ce qu'**il comporte au moins une vis de fixation (16), dont la tête d'extrémité comprend, depuis son extrémité libre jusqu'au corps de vis :
- une partie d'extrémité cylindrique (17), munie d'un filetage externe (18) complémentaire du filetage (14) aménagé sur la bordure interne de la collerette supérieure (7a) de la plaque support (2),
- une rainure annulaire (10) d'accueil de l'anneau élastique (8), et
- une protubérance annulaire (12),
l'épaulement annulaire supérieur (10b) de ladite rainure (10) étant adapté pour venir prendre appui sur la face en regard (8c) de l'anneau élastique (8), lui-même en appui par sa face (8d) contre la collerette inférieure (7b) de ladite plaque support (2), pour obtenir un verrouillage monobloc vis/plaque.

9. Dispositif orthopédique selon l'une quelconque des revendications 7 ou 8, **caractérisé en ce que** la bordure externe (8b) de l'anneau élastique (8) comporte un filetage (15) complémentaire de celui (14) de la collerette supérieure (7a) de la plaque support (2), de manière à autoriser le positionnement dudit anneau élastique (8) au sein de sa gorge de réception (5), après vissage au travers dudit filetage de plaque (14).

## Patentansprüche

1. Orthopädische Vorrichtung, im Besonderen in die Halswirbelsäule eines Patienten implantierbar, wobei die orthopädische Vorrichtung (1) sich einerseits aus einer Stützstruktur (2) vom Typ einer Platte, die durch eine Oberseite (2a) und eine Unterseite (2b) begrenzt ist und die mit wenigstens einem Durchgangsloch (3) versehen ist, und andererseits aus wenigstens einer aus einem Schraubenkopf (4a) und einem Schraubenkörper (4b) gebildeten Schraube (4) zusammensetzt, wobei das Durchgangsloch (3) so ausgebildet ist, daß die Schraube (4) hindurchgehen kann, wobei der Kontakt zwischen dem Schraubenkopf (4a) und der Platte (2) mittels komplementärer sphärischer Oberflächen (11', 7a') bewerkstelligt ist, wobei die orthopädische Vorrichtung (1) des Weiteren angesetzte Verriegelungsmittel aufweist, die geeignet sind, ein Zurückgehen der Schraube (4) zu verhindern oder zumindest zu begrenzen, sobald deren Kopf (4a) in geeigneter Weise auf der Platte (2) in Anlage ist, wobei die Verriegelungsmittel durch eine ringförmige, auf einem Teil der Höhe der inneren Oberfläche (6) des Lochs (3) ausgebildete, durch einen oberen ringförmigen Kragen (7a) und durch einen unteren ringförmigen Kragen (7b) begrenzte Nut (5) gebildet sind, wobei die Nut (5) durch eine obere ringförmige Schulter (5a), einen zylindrischen oder im Wesentlichen zylindrischen Nutboden (5b) und eine untere ringförmige Schulter (5c) begrenzt ist, wobei sich die obere (5a) und die untere (5c) Schulter senkrecht zur Achse des Lochs (3) erstrecken und deren Abstand (a) die Dicke der Nut (5) definiert, und wobei in die Nut (5) ein geschlitzter Metallring (8) mit radialer elastischer Verformungsfähigkeit eingebracht ist, bei dem im Ruhezustand einerseits der innere Rand (8a) einen Durchmesser aufweist, der kleiner als jener des Lochs (3) ist, und andererseits der äußere Rand (8b) einen Durchmesser aufweist, der kleiner als jener des Bodens (5b) der besagten ringförmigen Nut (5) ist, wobei die Dicke (b) des elastischen Rings (8) kleiner als die Dicke (a) der Nut (5), in der er untergebracht ist, ist, um ihm wenigstens einen axialen Freiheitsgrad im Inneren der Nut (5) zu geben, wobei der Schraubenkopf (4a) eine ringförmige Ausstülpung (12) aufweist, die sich vom Schraubenkörper (4b) aus bis zu einer ringförmigen, durch eine zur Achse der Schraube (4) senkrechte Schulter (10b), eine zylindrische oder im Wesentlichen zylindrische Bodenfläche (10a) und einen Abschnitt (10c) begrenzte Nut (10) erweitert, wobei die Ausstülpung (12) ausgebildet ist, um, wenn sich der besagte Kopf (4a) im zugehörigen Loch (3) voran bewegt, eine radiale Ausdehnung des elastischen Rings (8) in der diesen aufnehmenden Nut (5) und dann, in der Endphase des Schraubens, die Unterbringung des elastischen Rings (8) in der ringförmigen Nut (10) durch elastisches radiales Zusammenziehen zu ermöglichen, um so die besagten, ein Zurückgehen verhindernden Verriegelungsmittel zu bilden,
**dadurch gekennzeichnet, daß** :
a - das Loch (3) der Stützplatte (2) von der Oberseite (2a) der Platte (2) bis zu deren Unterseite (2b) nacheinander begrenzt ist durch:
- eine sphärische konkave, am inneren Rand des oberen Kragens (7a) gebildete Kontaktfläche (7a'),
- den inneren Rand des unteren Kragens (7b), wobei dieser innere Rand gegenüber dem Abschnitt der Schraube (4), der gegenüber kommen soll, überdimensioniert ist,
b - der Schraubenkopf (4a) von seinem freien Ende aus bis zum Schraubenkörper (4b) nacheinander aufweist:
b1 - eine sphärische konvexe, zur sphärischen konkaven Kontaktfläche (7a') der Stützplatte (2) komplementäre Kontaktfläche (11'),
b2 - die ringförmige, durch eine zur Achse der Schraube (4) senkrechte Schulter (10b), eine zylindrische oder im Wesentlichen zylindrische Bodenfläche (10a) und einen sich im Wesentlichen kegelstumpfförmig erweiternden Abschnitt (10c) begrenzte Nut (10), und
b3 - die am Schraubenkörper (4b) endende ringförmige Ausstülpung (12), wobei die ringförmige Ausstülpung (12) im Wesentlichen die Form eines sich vom Schraubenkörper (4b) bis zur besagten Nut (10) erweiternden Kegelstumpfs aufweist, wobei deren kleinster Durchmesser kleiner als jener des inneren Rands (8a) des elastischen Rings (8) und deren größter Durchmesser zwischen jenem des inneren Rands (8a) des Rings (8) und dem kleinsten Durchmesser des Lochs (3) liegt.

2. Orthopädische Vorrichtung gemäß Anspruch 1, **dadurch gekennzeichnet, daß** der Durchmesser des Bodens der ringförmigen Nut (5) des Lochs (3) wenigstens gleich dem um das Zweifache der Breite des zugehörigen elastischen Rings (8) vergrößerten größten Durchmesser der Ausstülpung (12) ist, wobei die Breite des Rings (8) dem Abstand zwischen dessen oberen (8a) und dessen unteren (8c) Rand entspricht.

3. Orthopädische Vorrichtung gemäß einem der Ansprüche 1 oder 2, **dadurch gekennzeichnet, daß** der elastische Ring (8) durch zwei ringförmige ebene parallele Oberflächen (8c) und (8d) begrenzt ist, deren Abstand die Dicke (b) des Rings (8) bestimmt.

4. Orthopädische Vorrichtung gemäß einem der Ansprüche 1 bis 3, **dadurch gekennzeichnet, daß** der elastische Ring (8) einen inneren Rand (8a) aufweist, dessen Durchmesser im Ruhezustand größer als jener des Bodens (10a) der ringförmigen Nut (10) des Schraubenkopfs (4a) ist.

5. Orthopädische Vorrichtung gemäß einem der Ansprüche 1 bis 4, **dadurch gekennzeichnet, daß** der elastische Ring (8) eine Dicke (b) aufweist, die kleiner als die Höhe der ringförmigen Nut (10) des Schraubenkopfs (4a) ist.

6. Orthopädische Vorrichtung gemäß einem der Ansprüche 1 bis 5, **dadurch gekennzeichnet, daß** der elastische Ring (8) einen inneren Rand (8a) aufweist, dessen Querschnitt im Wesentlichen eine nach innen zusammenlaufende V-Form aufweist, die zwei auf einander zu laufende Oberflächen bestimmt, und zwar eine obere (8e) und eine untere (8f).

7. Orthopädische Vorrichtung gemäß einem der Ansprüche 1 bis 6, **dadurch gekennzeichnet, daß** der innere Rand des oberen Kragens (7a) der Stützplatte (2) ein in der sphärischen konkaven Kontaktfläche (7a') geformtes zylindrisches Gewinde (14) aufweist.

8. Orthopädische Vorrichtung gemäß Anspruch 7, **dadurch gekennzeichnet, daß** sie wenigstens eine Befestigungsschraube (16) aufweist, deren Endkopf von dessen freien Ende aus bis zum Schraubenkörper folgendes aufweist:
- ein zylindrisches Endteil (17), das ein zum im inneren Rand des oberen Kragens (7a) der Stützplatte (2) geformten Gewinde (14) komplementäres Außengewinde (18) aufweist,
- eine ringförmige Nut (10) zur Aufnahme des elastischen Rings (8) und
- eine ringförmige Ausstülpung (12),
wobei die ringförmige obere Schulter (10b) der Nut (10) dazu ausgebildet ist, auf der dem elastischen Ring (8) gegenüberliegenden Fläche aufzuliegen, wobei der Ring selbst mit seiner Fläche (8d) am unteren Kragen (7b) der Stützplatte (2) anliegt, um eine einstückige Verriegelung Schraube/Platte zu erhalten.

9. Orthopädische Vorrichtung gemäß einem der Ansprüche 7 oder 8, **dadurch gekennzeichnet, daß** der äußere Rand (8b) des elastischen Rings (8) ein zu jenem (14) des oberen Kragens (7a) der Stützplatte (2) komplementäres Gewinde (15) aufweist, um ein Positionieren des elastischen Rings (8) in der Aufnahmenut (5) nach einem Schrauben durch das Plattengewinde (14) zu ermöglichen.

## Claims

1. Orthopaedic device, in particular implantable in the cervical spine of a patient, said orthopaedic device (1) comprising, on the one hand, a support structure (2) of the plate type, which is delimited by a top face (2a) and by a bottom face (2b), and which is provided with at least one through-orifice (3), and on the other hand, at least one screw (4) having a screw head (4a) and a screw body (4b), said through-orifice (3) being shaped so as to enable passage of said screw (4), the contact between said screw head (4a) and said plate (2) being made through complementary spherical surfaces (11', 7a'), said orthopaedic device (1) also comprising added locking means, able to prevent or at least to limit said screw (4) from moving back once its head (4a) suitably in rest on said plate (2), said locking means consisting of an annular groove (5), formed in a portion of the height of the internal surface (6) of said orifice (3), delimited by an upper annular flange (7a) and by a lower annular flange (7b), said groove (5) being delimited by an upper annular shoulder (5a), a cylindrical or substantially cylindrical bottom surface (5b) and a lower annular shoulder (5c), said upper (5a) and lower (5b) shoulders extending perpendicular to the axis of the orifice (3) and the distance (a) between them defining the thickness of said groove (5), and in which groove (5) is added a slotted metal ring (8) having a radial elastic deformation ability and of which, at rest, - on the one hand, the internal edge (8a) has a diameter smaller than that of said orifice (3), and - on the other hand, the external edge (8b) has a diameter smaller than that of the bottom (5b) of said annular groove (5), the thickness (b) of said spring ring (8) being smaller than the thickness (a) of said groove (5) in which it is accommodated, to be provided with at least one axial degree of freedom within said groove (5), said screw head (4a) comprising an annular protuberance (12) diverging from said screw body (4b) to an annular channel (10), delimited by a shoulder (10b) perpendicular to the axis of the screw (4), a cylindrical or substantially cylindrical bottom surface (10a), and a portion (10c), said protuberance (12) being shaped so as to enable, when said head (4a) moves forward within said associated orifice (3), radial expansion of said spring ring (8) within its receiving groove (5), and then, in the final phase of screwing, accommodation of said spring ring (8) within said annular channel (10), by elastic radial shrinkage, so as to form said backstop locking means,
**characterized in that**:
a- the orifice (3) of the support plate (2) is delimited by successively, from the top face (2a) of said plate (2) to the bottom face (2b) of the latter:
- a female spherical contact surface (7a') formed on the internal edge of said upper flange (7a),
- the internal edge of said lower flange (7b), this internal edge being oversized relative to the portion of said screw (4) that is intended to come opposite,
b- said screw head (4a) comprises successively, from its free end to said screw body:
b1- a male spherical contact surface (11'), complementary with the female spherical surface (7a') of the support plate (2),
b2- said annular channel (10) delimited by a shoulder (10b) perpendicular to the axis of the screw (4), a cylindrical or substantially cylindrical bottom surface (10a), and a generally diverging truncated portion (10c), and
b3- said annular protuberance (12) ending at said screw body (4b), said protuberance (12) has a generally diverging truncated shape from said screw body (4b) to said channel (10), with its minimum diameter smaller than that of the internal edge (8a) of said spring ring (8) and its maximum diameter comprised between that of said internal edge (8a) of said ring (8) and the minimum diameter of said orifice (3).

2. Orthopaedic device according to claim 1, **characterized in that** the bottom diameter of the annular groove (5) of the orifice (3) is at least equal to the maximum diameter of the protuberance (12), added with twice the width of the associated spring ring (8), said width of the ring (8) corresponding to the distance that separates the internal (8a) and external (8b) edges thereof.

3. Orthopaedic device according to any one of claims 1 or 2, **characterized in that** the spring ring (8) is delimited by two flat annular surfaces (8c) and (8d) and parallel to each other and the spacing of which defines the thickness (b) of said ring (8).

4. Orthopaedic device according to any one of claims 1 to 3, **characterized in that** the spring ring (8) has an internal edge (8a) whose diameter, at rest, is greater than that of the bottom (10a) of the annular channel (10) of the screw head (4a).

5. Orthopaedic device according to any one of claims 1 to 4, **characterized in that** the spring ring (8) has a thickness (b) smaller than the height of the annular channel (10) of the screw head (4a).

6. Orthopaedic device according to any one of claims 1 to 5, **characterized in that** the spring ring (8) comprises an internal edge (8a) with a generally V-shaped section converging inwards, defining two converging sloping surfaces, a top one (8e) and a bottom one (8f), respectively.

7. Orthopaedic device according to any one of claims 1 to 6, **characterized in that** the internal edge of the upper flange (7a) of the support plate (2) comprises a cylindrical thread (14) formed in the female spherical contact surface (7a').

8. Orthopaedic device according to claim 7, **characterized in that** it comprises at least one fixing screw (16), the end head of which comprises, from its free end to the screw body:
- a cylindrical end portion (17), provided with an external thread (18) complementary with the thread (14) formed on the internal edge of the upper flange (7a) of the support plate (2),
- an annular channel (10) for receiving the spring ring (8), and
- an annular protuberance (12),
the upper annular shoulder (10b) of said channel (10) being adapted to rest on the opposite face (8c) of the spring ring (8), the face (8d) of which rests against the lower flange (7b) of said support plate (2), to obtain an integral screw/plate locking.

9. Orthopaedic device according to any one of claims 7 or 8, **characterized in that** the external edge (8b) of the spring ring (8) comprises a thread (15) complementary with the thread (14) of the upper flange (7a) of the support plate (2), so as to allow the positioning of said spring ring (8) within its receiving groove (5), after screwing through said thread (14) of the plate.
